## Europäisches Patentamt
## European Patent Office
## Office européen des brevets

(11) Veröffentlichungsnummer: **0 197 174 B1**

# EUROPÄISCHE PATENTSCHRIFT

(45) Veröffentlichungstag der Patentschrift: **06.03.91**

(51) Int. Cl.5: **A47K 7/04**

(21) Anmeldenummer: **85104320.8**

(22) Anmeldetag: **10.04.85**

(54) **Waschautomat zur Reinigung von Gegenständen.**

(43) Veröffentlichungstag der Anmeldung:
**15.10.86 Patentblatt 86/42**

(45) Bekanntmachung des Hinweises auf die
Patenterteilung:
**06.03.91 Patentblatt 91/10**

(84) Benannte Vertragsstaaten:
**AT BE CH DE FR GB IT LI LU NL SE**

(56) Entgegenhaltungen:
**FR-A- 1 382 553**
**US-A- 3 220 424**

**MINI-MICRO SYSTEMS, Band 15, Nr. 11, November 1982, Seiten 201-211, Denver, Colorado, US; E. LUNDQUIST: "Robotic vision systems eye factory applications"**

(73) Patentinhaber: **Arzneimittel GmbH Apotheker
Vetter & Co. Ravensburg
Marienplatz 79-81
W-7980 Ravensburg(DE)**

(72) Erfinder: **Vetter, Helmut
Marienplatz 81
W-7980 Ravensburg(DE)**

(74) Vertreter: **Fay, Hermann, Dipl.-Phys. Dr. et al
Ensingerstrasse 21 Postfach 1767
W-7900 Ulm (Donau)(DE)**

## Beschreibung

Die Erfindung betrifft einen Waschautomaten zur Reinigung von Gegenständen, insbes. auch von Händen, mit einem im wesentlichen allseits geschlossenen Gehäuse, das zumindest eine Öffnung zur Einführung des zu reinigenden Gegenstandes aufweist, ferner mit mehreren, im Inneren des Gehäuses angeordneten, den Gegenstand mit flüssigen und/oder gasförmigen Reinigungsmedien beaufschlagenden Düsen, wobei die Düsen den zu reinigenden Flächen des Gegenstands entsprechend ausgerichtet bzw. ausrichtbar sind, ferner mit einer ebenfalls im Innern des Gehäuses angeordneten, auf die Lage des Gegenstandes im Gehäuse ansprechenden Kontrollvorrichtung, die bei für den Reinigungsvorgang vorgegebener Lage des Gegenstandes im Gehäuse den Reinigungsvorgang freigibt und mit einer den zeitlichen Ablauf des Reinigungsvorgangs steuernden und überwachenden Steuereinrichtung.

In zahlreichen industriellen Produktionsbereichen ebenso wie im medizinischen Bereich werben erhebliche Reinheitsanforderungen hinsichtlich Staubfreiheit bzw. Sterilität gestellt. Soweit solche Produktionsvorgänge sich vollständig in hermetisch dichten Räumen abspielen, stellt die Aufrechterhaltung der Reinheit im allgemeinen kein besonderes Problem dar. Kritisch ist jedoch stets das Einbringen von Gegenständen aus einer verunreinigten Umgebung in einen Reinraum, da hierbei erhebliche, dem Gegenstand anhaftende Verschmutzungen eingeschleppt werden. Hierzu bedarf es einer sorgfältigen und vollständigen Reinigung des Gegenstands.

Gleiches gilt für Arbeitskräfte, deren Anwesenheit in Reinräumen häufig unumgänglich ist. Zwar gibt es spezielle, beispielsweise staubabweisende Arbeitskleidung, durch die das Einschleppen von der Kleidung anhaftenden Verunreinigungen stark vermindert werden kann. Problematisch ist jedoch stets die Reinigung der mit den reinzuhaltenden Erzeugnissen in Berührung kommenden Hände, die häufig unsachgemäß durchgeführt wird, wodurch insbes. in der pharmazeutischen Produktion, in Kliniken, in der Lebensmittelverarbeitung und in ähnlichen Bereichen Keime in die Produktion oder auf reine Oberflächen übertragen werden. Solche beispielsweise in der Medizin und Pharmazie zu Infektionsgefahren und Produktionsausfällen führende, mangelhafte Reinigungsvorgänge sind in der Praxis schwer nachprüfbar und daher kaum abzustellen.

Ein Reinigungsgerät der eingangs genannten Art ist aus der US-A-3 220 434 bekannt. Um dort die Hände in eine für die Reinigung vorgesehene Stellung zu bringen und in dieser zu halten, sind an der Oberseite des Gehäuses am Rand der Öffnungen Führungsteile für die Hände vorgesehen, die aus flüssigkeitsdurchlässigem Material bestehen. Diese Führungsteile besitzen an ihrem der Öffnung zugewandten Ende eine im wesentlichen kreisförmige Querschnittsfläche, an dem entgegengesetzten Ende dagegen eine der Wand angepaßte ovale Querschnittsfläche. Dies wird durch ein an dem Führungsteil angeschlossenes ovales Rahmenteil erreicht, das zusätzlich mit quer zu seiner Längsöffnung sich erstreckenden Distanzstücken versehen ist, die bei in das Führungsteil eingeschobener Hand zwischen die einzelnen Finger treten. Auf diese Weise soll gewährleistet werden, daß die Reinigungsflüssigkeit auch zwischen die gespreizten Finger gelangen kann.

Bei diesem vorbekannten Reinigungsgerät für Hände wird die gewünschte Ausrichtung des zu reinigenden Gegenstandes durch mechanisch arbeitende Führungsteile erreicht. Dies hat zunächst den Nachteil, daß bei einem Einsatz des Waschautomaten für unterschiedlich geformte Gegenstände jeweils angepaßte Führungsteile eingesetzt werden müßten, was zu unvertretbar hohen Umrüstzeiten führen würde. Um dies zu vermeiden, könnte zwar für die zu reinigenden Gegenstände jeweils ein eigener Waschautomat vorgesehen werden, was aber ebenfalls einen nicht unbeträchtlichen Aufwand darstellen würde.

Darüber hinaus besitzt das mechanische Führungsteil aber auch den Nachteil, daß es den an sich erreichbaren Reinigungsgrad erheblich herabsetzt. Diese bekannte Reinigungseinrichtung ist daher nicht geeignet, die heutigen Anforderungen der Reinst-Technik zu erfüllen. Zum einen mindert das Führungsteil die Reinigungswirkung des von den Düsen abgegebenen und auf den zu reinigenden Gegenstand auftreffenden Reinigungsmittel nicht unerheblich ab. Darüber hinaus besteht die Gefahr, daß von dem zu reinigenden Gegenstand in das Führungsteil eingeschleppte größere Partikel oder sonstige Verschmutzungen sich in den flüssigkeitsdurchlässigen Materialdurchbrüchen des Führungsteils festsetzen und dann auf später zu reinigende Gegenstände übertragen werden könnten. Entscheidend für die Erreichung extrem hoher Reinheitsgrade ist es daher, daß jedwede Umhüllung des zu reinigenden Gegenstandes vermieden wird. Damit stellt sich aber andererseits das Problem, den zu reinigenden Gegenstand in die für seine Reinigung vorgesehene Position zu führen und sicher zu stellen, daß er in dieser verbleibt.

Der Erfindung liegt die Aufgabe zugrunde, einen Waschautomaten zur Reinigung von Gegenständen, insbes. auch von Händen zu schaffen, der den Reinigungsvorgang automatisiert, so daß der Reinigungsprozeß stets in der vorgeschriebenen, den jeweiligen Gegebenheiten angepaßten Weise abläuft und damit ein reproduzierbares Reinigungsergebnis erreicht wird.

Diese Aufgabe wird nach der Erfindung dadurch gelöst, daß die Kontrollvorrichtung entweder mehrere die Berührung mit dem zu reinigenden Gegenstand durch ein Ausgangssignal meldende Tastelemente in einer solchen Anzahl und Anordnung aufweist, daß eine Bewertungslogik von mehreren möglichen Ausrichtungen des zu reinigenden Gegenstands das Vorliegen der für den Reinigungsvorgang vorgesehenen Ausrichtung erkennt, oder eine optoelektronische Abtasteinrichtung aufweist, die den zu reinigenden Gegenstand linien- oder flächenhaft abtastet und das ermittelte Bildinformationssignal der Abtasteinrichtung einer Bewertungseinrichtung zuführt, die dieses auf Übereinstimmung mit einem erwarteten Bildmuster prüft, wobei die Bewertungslogik bzw. die Bewertungseinrichtung bei Vorliegen einer vorgegebenen Form bzw. Gestalt des Gegenstandes und/oder seiner für die Reinigung vorgeschriebenen Ausrichtung ein den Ablauf des Reinigungsvorgangs beeinflussendes Freigabesignal an die Steuereinrichtung leitet.

Der durch die Erfindung erreichte Fortschritt besteht im wesentlichen darin, daß der Reinigungsvorgang nur dann abläuft, wenn der Gegenstand die für die optimale Reinigung vorgesehene Lage im Gehäuse eingenommen hat, so daß die wirksame Reinigung aller Flächen erfolgen kann. Dabei kann, wenn der Waschautomat zur Reinigung von Händen vorgesehen ist, die Anordnung der Tastelemente so gewählt sein, daß diese nur mit gespreizten Fingern berührt werden können. Dadurch ist sicher gestellt, daß eine hinreichende Reinigung auch der Fingerzwischenräume erfolgt. Sofern bei der Verwendung von Tastelementen die Reinigungswirkung im Bereich der gegenseitigen Berührungsflächen u. U. eingeschränkt sein kann, wird durch die völlig berührungsfreie Überwachung des zu reinigenden Gegenstands bezüglich seiner Gestalt und Ausrichtung erreicht, daß der Reinigungsvorgang uneingeschränkt an der gesamten Oberfläche des Gegenstands ablaufen kann.

Die Auswahl und Dosierung der dabei zur Anwendung kommenden Reinigungsmittel, sowie die Reihenfolge und die zeitliche Dauer ihrer Anwendung richten sich nach einem fest vorgegebenen Programmablauf. Hierauf hat der Bediener des Gerätes, soweit es sich um die Reinigung von Gegenständen handelt, bzw. der Benutzer des Gerätes, der darin seine Hände reinigt, keinen Einfluß. Der Waschautomat kann durch Änderung des Programmablaufs auch ohne weiteres an unterschiedliche Anforderungen angepaßt werden. Damit werden insgesamt eine von personellen Gewohnheiten und Unzulänglichkeiten unabhängige hohe Reproduzierbarkeit und die im pharmazeutischen Bereich erforderliche Validierung gewährleistet.

In bevorzugter Ausführungsform der Erfindung ist die Öffnung mit einer randseitig angeschlossenen Manschette zur Abdichtung gegen den zu reinigenden Gegenstand versehen. Somit wird während des Reinigungsvorgangs eine vollständige Abdichtung des Gehäuses auch im Bereich der Öffnungen erreicht, wodurch einerseits keine Verschmutzungen oder Keime in das Innere des Waschautomaten eindringen können und andererseits Reinigungsflüssigkeit nicht nach außen gelangen kann. Um ein besseres Anschmiegen der Manschette zu erreichen, ist es von Vorteil, wenn die Manschette einen ringförmigen, im wesentlichen in Umfangsrichtung der Öffnung verlaufenden und zur dichten Anlage am zu reinigenden Gegenstand mit Druck beaufschlagbaren Hohlraum aufweist. Unter Druckbeaufschlagung kann sich daher die Manschette dem durch die Öffnung ins Innere des Automaten sich erstreckenden Gegenstands dichtend anlegen. Sofern es sich hierbei um einen Waschautomaten zur Reinigung von Händen handelt, wird die Druckbeaufschlagung so gewählt, daß eine ausreichende Abdichtung gegeben ist, dennoch der Unterarm nicht zu stark belastet wird, so daß in Notfällen auch die Möglichkeit besteht, die Hand bei druckbeaufschlagter Manschette aus dem Gerät herauszuziehen.

In einer für besonders hohe Reinheitsanforderungen vorgesehenen Ausführungsform der Erfindung ist das Gehäuse als druckdichtschließendes und druckbeaufschlagbares Druckgefäß ausgebildet und dazu mit Schließeinrichtungen an der bzw. den Öffnungen sowie mit einem Druckanschluß zur Zufuhr von Reindampf versehen. Dadurch kann der Waschautomat zur gelegentlichen innenseitigen Sterilisation z. B. mit strömendem Reindampf von 121 °C thermisch antimikrobiell behandelt werden.

Das Gehäuse besteht zweckmäßigerweise aus einem gegen Reinigungsmedien resistenten und inerten Material wie Edelstahl. Die Oberseite des Gehäuses kann dabei vorteilhafterweise von durchsichtigem Material wie Makrolon gebildet sein, so daß der Ablauf des Reinigungsvorganges beobachtet werden kann.

Für einen besonders kompakten Aufbau des Waschautomaten empfiehlt es sich, daß für die Reinigungsmedien vorgesehene Vorratsbehälter; ferner die Anwendung und Auswahl der verschiedenen Reinigungsmedien tätigende Steuerungs- und Regelelemente sowie die Vorratsbehälter mit den Steuerungs- und Regelelementen und den Düsen verbindende Armaturen innerhalb des Gehäuses angeordnet sind. Es besteht jedoch ebenso die Möglichkeit, zumindest die Vorratsbehälter außerhalb des Waschautomaten anzuordnen, wodurch die Nachfüllung der Reinigungs- und Desinfektionslösungen erleichtert wird. Dabei kann insbes. auch jeweils ein Vorratsbehälter der Versorgung von mehreren Waschautomaten dienen.

In einer weiter bevorzugten Ausführungsform der Erfindung ist die Steuereinrichtung mit einer Personalerfassungseinrichtung in Form einer numerischen Code-Eingabetastatur oder einem Leser für Ausweiskarten versehen. Dadurch besteht die Möglichkeit, den einzelnen Benutzer zu identifizieren und beispielsweise Uhrzeit und Häufigkeit der Reinigungsvorgänge des einzelnen Benutzers zu erfassen. Schließlich ist es von Vorteil, wenn die Steuereinrichtung ein Bewertungssignal erzeugt, das die erfolgte Durchführung des vorgesehenen Reinigungsvorgangs anzeigt. Dieses Bewertungssignal kann z. B. dem Benutzer anzeigen, daß der vorgesehene Reinigungsvorgang vorschriftsmäßig abgelaufen ist. Ebenso kann das Bewertungssignal für eine Türsteuerung dienen, so daß beispielsweise ein Reinraum nur nach einem zuvor vorschriftsmäßig abgelaufenen Reinigungsvorgang betreten werden kann.

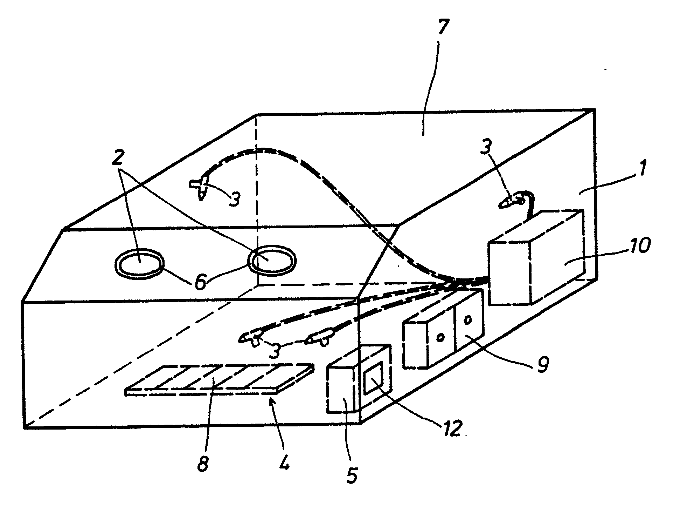

Im folgenden wird die Erfindung an einem in der Zeichnung dargestellten Ausführungsbeispiel näher erläutert; die einzige Figur zeigt den Waschautomaten nach der Erfindung in teilweise nur schematischer Darstellung.

Der Waschautomat dient allgemein zur Reinigung von Gegenständen, ißt jedoch in der in der Zeichnung dargestellten Weise insbes. zur Reinigung von Händen vorgesehen. Dazu weist er ein im wesentlichen allseits geschlossenes Gehäuse 1 auf, das mit zwei Öffnungen 2 versehen ist, durch die die zu reinigenden Hände ins Innere des Waschautomaten eingeführt werden. Im Inneren des Gehäuses 1 sind mehrere Düsen 3 zur Abgabe von flüssigen und gegebenenfalls auch gasförmigen Reinigungsmedien angeordnet. Die Düsen 3 sind im einzelnen so ausgerichtet bzw. ausrichtbar, daß alle zu reinigenden Flächen der Hände erfaßt werden.

Im Inneren des Gehäuses 1 ist ferner eine Kontrollvorrichtung 4 angeordnet, die auf die Form und die Lage des zu reinigenden Gegenstands im Gehäuse 1 anspricht. Sofern diese mit der für die Reinigung vorgesehenen übereinstimmt, wird der Reinigungs vorgang durch eine Steuereinrichtung 5 freigegeben, die im übrigen auch den verfahrensmäßigen und/oder zeitlichen Ablauf des Reinigungsvorgangs steuert und überwacht.

Die Öffnung 2 ist mit einer randseitig angeschlossenen elastischen Manschette 6 zur Abdichtung gegen den zu reinigenden Gegenstand versehen. Dadurch wird verhindert, daß Reinigungsflüssigkeit aus dem Inneren des Gehäuses 1 nach außen gelangen kann. Ebenso wird das Eindringen von Staub und Keimen in das Gehäuse 1 während des Reinigungsvorgangs unterbunden. Dabei kann die Manschette 6 in in der Zeichnung nicht dargestellter Weise einen ringförmigen, im wesentlichen in Umfangsrichtung der Öffnung verlaufenden und zur dichten Anlage am zu reinigenden Gegenstand mit Druck beaufschlagbaren Hohlraum aufweisen. Auf diese Weise ist ein leichtes Einführen des Gegenstands in den Waschautomaten möglich, da die Manschette 6 sich diesem erst zu Beginn des Reinigungsvorgangs dichtend anlegt.

Das Gehäuse 1 kann in ebenfalls nicht näher dargestellter Weise als druckdichtschließendes und druckbeaufschlagbares Druckgefäß ausgebildet sein, um gelegentlich eine thermisch antimikrobielle Behandlung des Innenraums z. B. mit strömendem Reindampf bei einer Temperatur von 121° C durchführen zu können. Dazu ist das Gehäuse 1 mit im einzelnen nicht dargestellten Schließeinrichtungen an den Öffnungen 2 sowie mit einem Druckanschluß zur Zufuhr von Reindampf versehen. Das Gehäuse 1 besteht aus einem gegen Reinigungsmedien resistenten und inerten Material wie beispielsweise Edelstahl, wobei die Oberseite 7 des Gehäuses 1 zur Beobachtung des Reinigungsvorgangs von durchsichtigem Material wie beispielsweise Makrolon gebildet ist.

Die Kontrollvorrichtung 4 weist eine optoelektronische Abtasteinrichtung 8 auf, die den zu reinigenden Gegenstand je nach dessen Gestalt beispielsweise linien- oder flächenhaft abtastet. Das so ermittelte Bildinformationssignal wird einer Bewertungseinrichtung zugeführt, die bei Vorliegen eines erwarteten Bildmusters ein den Ablauf des Reinigungsvorgangs ermöglichendes Freigabesignal an die Steuereinrichtung 5 leitet. Derartige optisch arbeitende und erkennende Kontrollvorrichtungen sind bekannt, wofür beispielsweise die DE-C-26 38 138 genannt werden kann, in welcher eine solche Kontrollvorrichtung zum Erkennen und Aussortieren fehlerhafter Packungen beschrieben ist. In einer einfacheren Ausführungsform kann die Kontrollvorrichtung in in der Zeichnung nicht näher dargestellter Weise auch Tastelemente aufweisen, die bei Berührung mit dem zu reinigenden Gegenstand jeweils ein Ausgangssignal erzeugen. Diese Ausgangssignale können dann einer Bewertungslogik zugeführt werden, die wiederum bei Vorliegen der vorgegebenen Form und Gestalt ein den Ablauf des Reinigungsvorgangs ermöglichendes Freigabesignal an die Steuereinrichtung 5 leitet. Bei einem zur Reinigung von Händen vorgesehenen Waschautomaten wird die Kontrollvorrichtung so eingerichtet sein, daß eine Freigabe des Waschvorgangs nur dann erfolgt, wenn die einzelnen Finger gegenseitig abgespreizt sind, so daß auch die Fingerzwischenräume der Reinigung unterworfen werden.

Bei der in der Zeichnung dargestellten kompakten Ausbildung des Waschautomaten sind die Reinigungs- und Desinfektionslösungen enthaltende Vorratsbehälter 9, ferner die Anwendung und Auswahl der verschiedenen Reinigungsmedien tätigende Steuerungs- und Regelelemente 10, also beispielsweise Dosierpumpen, Ventile und dergl. und schließlich auch die Vorratsbehälter 9 mit den Steuerungs- und Regelelementen 10 und Düsen 3 verbindenden Armaturen 11 innerhalb des Gehäuses 1 angeordnet. Jedoch besteht ebenso die Möglichkeit, mehrere solcher Waschautomaten an jeweils nur einen zentral aufgestellten Vorratsbehälter 9 anzuschließen, wodurch insbes. eine einfachere Nachfüllung der Reinigungs- und Desinfektionslösungen möglich ist.

Schließlich ist die Steuereinrichtung noch mit einer nur angedeuteten Personalerfassungseinrichtung in Form eines Lesers 12 für Ausweiskarten versehen, der Bestandteil der Steuereinrichtung 5 ist. Dadurch läßt sich beispielsweise die Häufigkeit und der Zeitpunkt von Reinigungsvorgängen für jeden einzelnen Benutzer erfassen. Dabei kann die Steuereinrichtung 5 auch ein Bewertungssignal erzeugen, das als Quittierung eines ordnungsgemäß abgelaufenen Reinigungsvorgangs für den Benutzer dienen kann. Das Bewertungssignal kann jedoch auch zur Steuerung des Zugangs zu einem Reinraum in der Weise verwendet werden, daß sich beispielsweise die Tür nur nach einem vorschriftsmäßig abgelaufenen Reinigungsvorgang öffnen läßt.

Der Waschautomat kann im übrigen auch so eingerichtet sein, daß sich im Anschluß an die Reinigung ein Trocknungsvorgang anschließt, indem über die Düsen sterile Luft ins Innere des Gehäuses geblasen wird.

Die Steuereinrichtung ist zweckmäßigerweise bezüglich des verfahrensmäßigen und zeitlichen Ablaufs des Reinigungsvorgangs frei programmierbar. So kann beispielsweise folgender Reinigungsablauf vorgegeben werden:

Nach Erfassung der Personaldaten werden das Vorliegen der vorgegebenen Form und Lage überprüft und bei Übereinstimmung die Manschetten 6 zur Anlage druckbeaufschlagt. Nach einer Kurzbedüsung zur Benetzung erfolgt der Hauptreinigungsgang, dem sich ein Nachspülen mit klarem Wasser und ein Trocknungsvorgang mit Sterilluft anschließen. Nach einer Bedüsung mit Desinfektionslösung und einer Registrierung des neu gemessenen Status zur quantitativen Beurteilung des Reinigungsergebnisses erfolgt die Öffnung der Manschette 6 durch Druckentlastung und gegebenenfalls die Freigabe nachgeordneter Schleusentüren sowie die vorübergehende Öffnung des Abwasserablaufventils.

## Ansprüche

1. Waschautomat zur Reinigung von Gegenständen, insbes. auch von Händen, mit einem im wesentlichen allseits geschlossenen Gehäuse (1), das zumindest eine Öffnung (2) zur Einführung des zu reinigenden Gegenstandes aufweist, ferner mit mehreren, im Inneren des Gehäuses (1) angeordneten, den Gegenstand mit flüssigen und/oder gasförmigen Reinigungsmedien beaufschlagenden Düsen (3), wobei die Düsen (3) den zu reinigenden Flächen des Gegenstands entsprechend ausgerichtet bzw. ausrichtbar sind, ferner mit einer ebenfalls im Innern des Gehäuses (1) angeordneten, auf die Lage des Gegenstandes im Gehäuse ansprechenden Kontrollvorrichtung (4), die bei für den Reinigungsvorgang vorgegebener Lage des Gegenstandes im Gehäuse (1) den Reinigungsvorgang freigibt und mit einer den zeitlichen Ablauf des Reinigungsvorgangs steuernden und überwachenden Steuereinrichtung (5), dadurch gekennzeichnet, daß die Kontrollvorrichtung entweder mehrere die Berührung mit dem zu reinigenden Gegenstand durch ein Ausgangssignal meldende Tastelemente in einer solchen Anzahl und Anordnung aufweist, daß eine Bewertungslogik von mehreren möglichen Ausrichtungen des zu reinigenden Gegenstands das Vorliegen der für den Reinigungsvorgang vorgesehenen Ausrichtung erkennt, oder eine optoelektronische Abtasteinrichtung (8) aufweist, die den zu reinigenden Gegenstand linien- oder flächenhaft abtastet und das ermittelte Bildinformationssignal der Abtasteinrichtung (8) einer Bewertungseinrichtung zuführt, die dieses auf Übereinstimmung mit einem erwarteten Bildmuster prüft, wobei die Bewertungslogik bzw. die Bewertungseinrichtung bei Vorliegen einer vorgegebenen Form bzw. Gestalt des Gegenstandes und/oder seiner für die Reinigung vorgeschriebenen Ausrichtung ein den Ablauf des Reinigungsvorgangs beeinflussendes Freigabesignal an die Steuereinrichtung (5) leitet.

2. Waschautomat nach Anspruch 1, dadurch gekennzeichnet, daß die Öffnung (2) mit einer randseitig angeschlossenen elastischen Manschette (6) zur Abdichtung gegen den zu reinigenden Gegenstand versehen ist.

3. Waschautomat nach Anspruch 2, dadurch gekennzeichnet, daß die Manschette (6) einen ringförmigen, im wesentlichen in Umfangsrichtung der Öffnung (2) verlaufenden und zur dichten Anlage am zu reinigenden Gegenstand mit Druck beaufschlagbaren Hohlraum aufweist.

4. Waschautomat nach den Ansprüchen 1 bis 3, dadurch gekennzeichnet, daß das Gehäuse (1) als druckdicht schließendes und druckbeaufschlagbares Druckgefäß ausgebildet ist und dazu mit Schließeinrichtungen an der bzw. den Öffnungen sowie mit einem Druckanschluß zur Zufuhr von Reindampf versehen ist.

5. Waschautomat nach den Ansprüchen 1 bis 4, dadurch gekennzeichnet, daß das Gehäuse (1) aus einem gegen Reinigungsmedien resistenten und inerten Material wie Edelstahl besteht.

6. Waschautomat nach den Ansprüchen 1 bis 5, dadurch gekennzeichnet, daß die Oberseite (7) des Gehäuses (1) von durchsichtigem Material wie Makrolon gebildet ist.

7. Waschautomat nach den Ansprüchen 1 bis 6, dadurch gekennzeichnet, daß für die Reinigungsmedien vorgesehene Vorratsbehälter (9), ferner die Anwendung und Auswahl der verschiedenen Reinigungsmedien tätigende Steuerungs- und Regelelemente (10) sowie die Vorratsbehälter (9) mit den Steuerungs- und Regelelementen (10) und den Düsen (3) verbindende Armaturen (11) innerhalb des Gehäuses (1) angeordnet sind.

8. Waschautomat nach den Ansprüchen 1 bis 7 , dadurch gekennzeichnet, daß die Steuereinrichtung (5) mit einer Personalerfassungseinrichtung in Form einer numerischen Code-Eingabetastatur oder einem Leser (12) für Ausweiskarten versehen ist.

9. Waschautomat nach den Ansprüchen 1 bis 8 , dadurch gekennzeichnet, daß die Steuereinrichtung (5) ein Bewertungssignal erzeugt, das die erfolgte Durchführung des vorgesehenen Reinigungsvorgangs anzeigt.

## Claims

1. An automatic washer for cleaning items, in particular also hands, comprising a housing (1) which is closed substantially on all sides and which has at least one opening (2) for introduction of the item to be cleaned, further comprising a plurality of nozzles (3) which are disposed in the interior of the housing (1) and which act on the item with liquid and/or gaseous cleaning agents, wherein the nozzles (3) are or can be oriented to correspond to the surfaces to be cleaned on the item, further comprising a monitoring device (4) which is also disposed in the interior of the housing (1) and which responds to the position of the item in the housing and which enables the cleaning operation when the item is in a predetermined position in the housing (1) for the cleaning operation, and further comprising a control device (5) for controlling and monitoring the procedure in respect of time of the cleaning operation, characterised in that either the monitoring means has such a number and arrangement of a plurality of sensing elements which by means of an output signal signal contact with the item to be cleaned, that out of a plurality of possible orientations of the item to be cleaned an evaluation logic means recognises the existence of the orientation intended for the cleaning operation, or the monitoring device has an opto-electronic scanning means (8) which scans the item to be cleaned in a line or surface mode and passes the detected image information signal of the scanning means (8) to an evaluation means which checks said signal for coincidence with an expected image pattern, wherein when there is a predetermined form or configuration of the item and/or when it is in the orientation prescribed for the cleaning procedure, the evaluation logic means or the evaluation means passes to the control means (5) an enable signal for influencing the procedure involved in the cleaning operation.

2. An automatic washer according to claim 1 characterised in that the opening (2) is provided with a resilient cuff (6) connected thereto at the edge for sealing relative to the item to be cleaned.

3. An automatic washer according to claim 2 characterised in that the cuff (6) has an annular hollow space which extends substantially in the peripheral direction of the opening (2) and which can be subjected to pressure to provide for sealing contact against the item to be cleaned.

4. An automatic washer according to claims 1 to 3 characterised in that the housing (1) is in the form of a pressure-tightly closing pressure vessel which can be subjected to pressure and for that purpose is provided with closing means at the opening or openings and with a pressure connection for the supply of clean steam.

5. An automatic washer according to claims 1 to 4 characterised in that the housing (1) comprises a material which is resistant to cleaning agents and inert, such as high-quality steel.

6. An automatic washer according to claims 1 to 5 characterised in that the top side (7) of the housing (1) is formed by transparent material such as Makrolon.

7. An automatic washer according to claims 1 to 6 characterised in that supply containers (9) provided for the cleaning agents, and also control and regulating elements (10) for dealing with the use and selection of the various cleaning agents, as well as fittings (11) connecting the supply containers (9) to the control and regulating elements (10) and the nozzles (3) are disposed within the housing (1).

8. An automatic washer according to claims 1 to 7 characterized in that the control device (5) is provided with a personnel detection means in the form of a numerical code input keyboard or a reader (12) for identity cards.

9. An automatic washer according to claims 1 to 8 characterised in that the control means (5) produces an evaluation signal which indicates that the intended cleaning operation has been carried out.

**Revendications**

1. Appareil de lavage automatique servant à nettoyer des objets, et notamment également les mains, comportant un boîtier (1) sensiblement fermé de tous côtés, qui comporte au moins une ouverture (2) permettant d'introduire l'objet à nettoyer, et en outre plusieurs buses (3) disposées à l'intérieur du boîtier (1) et envoyant à l'objet des milieux de nettoyage liquides et/ou gazeux, les buses (3) étant dirigées ou pouvant être dirigées en fonction des surfaces à nettoyer de l'objet, et comportant en outre un dispositif de contrôle (4) qui est disposé également à l'intérieur du boîtier (1), répond à la position de l'objet dans le boîtier et déclenche l'opération de nettoyage dans le cas où l'objet se trouve dans une position prédéterminée pour l'opération de nettoyage à l'intérieur du boîtier (1), et un dispositif de commande (5) qui commande et contrôle le déroulement dans le temps de l'opération de nettoyage, caractérisé en ce que le dispositif de contrôle comporte soit plusieurs éléments de détection signalant par un signal de sortie le contact avec l'objet à nettoyer et prévus en un nombre tel et selon une disposition telle qu'un système logique d'évaluation identifie, parmi plusieurs orientations possibles de l'objet à nettoyer, la présence de l'orientation prévue pour l'opération de nettoyage, ou possède un dispositif d'exploration opto-électronique (8), qui explore ligne-par-ligne ou selon des surfaces l'objet à nettoyer et envoie le signal déterminé d'information d'image du dispositif d'exploration (8) à un dispositif d'évaluation, qui contrôle ce signal d'information d'image pour savoir s'il coïncide avec un profil d'image attendu, auquel cas, lors de la présence d'une forme ou configuration prédéterminée de l'objet et/ou de son alignement prescrit pour le nettoyage, le système logique d'évaluation ou le dispositif d'évaluation envoie au dispositif de commande (5) un signal de libération qui influe sur le déroulement de l'opération de nettoyage.

2. Appareil de lavage automatique selon la revendication 1, caractérisé en ce que l'ouverture (2) comporte un manchon élastique (6) raccordé au bord de l'ouverture et servant à établir une étanchéité vis-à-vis de l'objet à nettoyer.

3. Appareil de lavage automatique selon la revendication 2, caractérisé en ce que le manchon (6) possède une cavité annulaire, qui s'étend sensiblement dans la direction circonférentielle de l'ouverture (2) et peut être chargée par une pression pour permettre une application étanche contre l'objet à nettoyer.

4. Appareil de lavage automatique selon les revendications 1 à 3, caractérisé en ce que le boîtier (1) est réalisé sous la forme d'une enceinte sous pression, qui se ferme d'une manière étanche à la pression et peut être chargée par une pression, et comporte à cet effet des dispositifs de fermeture au niveau de la ou des ouvertures ainsi qu'un raccord de pression pour l'envoi d'une vapeur pure.

5. Appareil de lavage automatique selon les revendications 1 à 4, caractérisé en ce que le boîtier (1) est constitué par un matériau résistant vis-à-vis des milieux de nettoyage et inerte, comme par exemple un acier traité.

6. Appareil de lavage automatique selon les revendications 1 à 5, caractérisé en ce que la face supérieure (7) du boîtier (1) est formée par un matériau transparent, comme par exemple du Makrolon.

7. Appareil de lavage selon les revendications 1 à 6, caractérisé en ce que des réservoirs (9) pour les milieux de nettoyage et en outre des organes de commande et de réglage (10) déclenchant l'utilisation et le choix des différents milieux de nettoyage ainsi que des éléments de robinetterie (11) reliant les réservoirs (9) aux organes de commande et de réglage (10) et aux buses (3) sont disposés à l'intérieur du boîtier (1).

8. Appareil de lavage automatique selon les revendications 1 à 7, caractérisé en ce que le dispositif de commande (5) comporte un dispositif d'identification personnelle se présentant sous la forme d'un clavier numérique d'entrée d'un code ou d'un lecteur (12) pour des cartes d'identité.

9. Appareil de lavage automatique selon les revendications 1 à 8, caractérisé en ce que le dispositif de commande (5) produit un signal d'évaluation, qui indique l'exécution effective de l'opération prévue de nettoyage.